# EUROPÄISCHE PATENTSCHRIFT

(11) **EP 0 801 683 B1**
(45) Veröffentlichungstag und Bekanntmachung des Hinweises auf die Patenterteilung: **02.05.2002**
(21) Anmeldenummer: 96900601.4
(22) Anmeldetag: 20.01.1996
(51) Int. Cl.: C12P 13/02, C12P 41/00

(54) **RACEMATSPALTUNG PRIMÄRER UND SEKUNDÄRER HETEROATOMSUBSTITUIERTER AMINE DURCH ENZYM-KATALYSIERTE ACYLIERUNG**
RACEMATE SEPARATION OF PRIMARY AND SECONDARY HETEROATOM-SUBSTITUTED AMINE BY ENZYME-CATALYSED ACYLATION
SEPARATION DES RACEMATES D'AMINES PRIMAIRES ET SECONDAIRES SUBSTITUEES PAR DES HETEROATOMES PAR ACYLATION CATALYSEE PAR DES ENZYMES

(30) Priorität: 03.02.1995 DE 19503605; 29.06.1995 DE 19523151
(43) Veröffentlichungstag der Anmeldung: 22.10.1997
(73) Patentinhaber: BASF AKTIENGESELLSCHAFT, 67056 Ludwigshafen (DE)
(72) Erfinder: BALKENHOHL, Friedhelm, 67117 Limburgerhof (DE); DITRICH, Klaus, 67161 Gönnheim (DE); NÜBLING, Christoph, 67454 Ha loch (DE)
(86) Internationale Anmeldenummer: EP9600234
(87) Internationale Veröffentlichungsnummer: WO9623894

(56) Entgegenhaltungen:
- DE-A- 4 332 738
- US-A- 5 057 607
- J. CHEM. SOC., PERKIN TRANS. 1 (1993), (20), 2453-6 CODEN: JCPRB4;ISSN: 0300-922X, XP002000855 GOTOR, VICENTE ET AL: "Synthesis of optically active amides from.beta.-furyl and.beta.-phenyl esters by way of enzymic aminolysis"
- TETRAHEDRON LETT. (1991), 32(33), 4197-8 CODEN: TELEAY;ISSN: 0040-4039, XP002000856 ASENSIO, GREGORIO ET AL: "Enzyme-mediated enantioselective acylation of secondary amines in organic solvents"
- JOURNAL OF THE CHEMICAL SOCIETY, CHEMICAL COMMUNICATIONS, Nr. 14, 15.Juli 1988, LETCHWORTH GB, Seiten 957-958, XP002000857 VICENTE GOTOR ET AL.: "Enantioselective acylation of amino alcohols by porcine pancreatic lipase"

## Beschreibung

Die vorliegende Erfindung betrifft ein neues Verfahren zur Racematspaltung primärer und sekundärer heteroatomsubstituierter Amine durch Umsetzung mit einem Ester in Gegenwart einer Hydrolase und anschließende Trennung des einen enantioselektiv acylierten heteroatomsubstituierten Amins vom nicht umgesetzten anderen Enantiomer des heteroatomsubstituierten Amins.

In der nach Prioritätstag der vorliegenden Erfindung veröffentlichten Schrift WO 95/08636 wird ein Verfahren zur Racematspaltung primärer und sekundärer Amine durch Umsetzung mit einem Ester in Gegenwart einer Hydrolase beschrieben. Als bevorzugte Amine werden dort primäre Arylalkylamine genannt. Es findet sich jedoch kein Hinweis auf die Verwendbarkeit von heteroatomsubstituierten Aminen.

Überraschenderweise wurde nun gefunden, daß das eingangs beschriebene Verfahren besonders vorteilhaft funktioniert, wenn als heteroatomsubstituiertes Amin ein Amin der allgemeinen Formel I 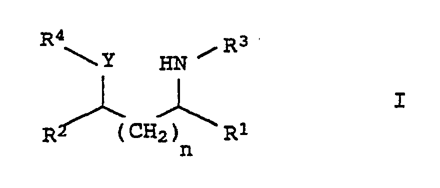 wobei
- n: 0, 1, 2, 3;
- Y: O, S, NH, NR⁵;
- R¹, R²: unabhängig voneinander jeweils H, Alkyl oder Aryl oder R¹ und R² oder R² und R³, oder R¹ und R⁴ zusammen mit den benachbarten C-Atomen Teil eines Ringsystems;
- R⁴: Alkyl oder Arylalkyl;
- R³,R⁵: unabhängig voneinander H, Alkyl oder Arylalkyl bedeuten,
eingesetzt wird.

Weiterhin wurde ein Verfahren zur Herstellung von acylierten primären und sekundären Aminen durch Umsetzung der heteroatomsubstituierten Amine mit einem Ester unter spezifischer Katalyse mit einer Lipase ausgewählt aus der Gruppe SP 523, SP 524, SP 525, SP 526 oder Novozym® 435 gefunden, das dadurch gekennzeichnet ist, daß die Säurekomponente des Esters ein Sauerstoffatom in Nachbarschaft des Carbonylkohlenstoffatoms trägt.

Die für das erfindungsgemäße Verfahren geeigneten Ester sind solche, die in der Säurekomponente des Esters ein elektronenreiches Heteroatom in Nachbarschaft zum Carbonylkohlenstoff tragen oder bei denen in der Säurekomponente ein Akzeptorsubstituent in Form eines oder mehrerer Heteroatome in Nachbarschaft des Carbonylkohlenstoffatoms sitzt.

Das Heteroatom muß über mindestens ein freies Elektronenpaar verfügen. Es soll sich in der Nachbarschaft zum Carbonylkohlenstoff befinden. Darunter ist die Bindung des Heteroatoms an ein Kohlenstoffatom in alpha-Position zum Carbonylkohlenstoff gemeint. Das Heteroatom kann auch eine Mehrfachbindung zum Kohlenstoff aufweisen, wie sie in der Cyanogruppe vorkommt. Als Heteroatom ist Sauerstoff bevorzugt.

Das Heteroatom kann gegebenenfalls mit weiteren Gruppen, z.B. Alkylgruppen, verknüpft sein. Ist das Heteroatom beispielsweise Sauerstoff, so liegt eine Ethergruppe vor.

Besonders geeignete Ester sind solche mit der Struktur 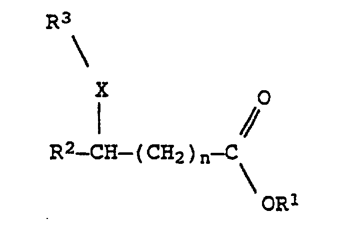 worin
- R¹: = C₁-C₁₀-Alkyl,
- R²: = C₁-C₁₀-Alkyl, H
- R³: = H, C₁-C₁₀-Alkyl, gegebenenfalls durch NH₂, OH, C₁-₄-Alkoxy oder Halogen substituiertes Phenyl,
- X: = O
- n: = 0
bedeuten. Unter diesen sind die C₁-₄-Alkylester der C₁-₄-Alkoxyessigsäuren, wie der Methoxyessigsäureethylester, bevorzugt.

Die von Novo Nordisk (Enzyme Toolbox) kommerziell erhältlichen Lipasen SP 523, SP 524, SP 525, SP 526 und Novozym® 435 sind mikrobielle Lipasen, die aus Hefen wie Candida antarctica herstellbar sind.

Des weiteren eignen sich die Lipasen "Chirazyme L1 bis L8', welche kommerziell erhältlich sind (Boehringer Mannheim), für das erfindungsgemäße Verfahren.

Das verwendete Enzym kann in nativer oder in immobilisierter Form eingesetzt werden. Besonders gut eignet sich das immobilisierte Enzym Novozym® 435.

Die erfindungsgemäßen Verfahren können unter Verwendung von Lösungsmitteln oder auch lösungsmittelfrei durchgeführt werden.

Als Lösungsmittel sind generell organische Lösungsmittel geeignet. Besonders gut verläuft die Reaktion in Ethern, beispielsweise in MTBE 1,4-Dioxan oder THF, oder in Kohlenwasserstoffen wie Hexan, Cyclohexan, Toluol oder halogenierten Kohlenwasserstoffen wie Methylenchlorid.

Die Umsetzung des Esters mit dem racemischen heteroatomsubstituierten Amin unter Enzymkatalyse wird üblicherweise bei Raumtemperatur ausgeführt. Die Reaktionszeiten dafür betragen je nach Substrat 1 bis 48 Stunden. Sekundäre heteroatomsubstituierte Amine benötigen in der Regel längere Reaktionszeiten als primäre heteroatomsubstituierte Amine. Die geringere Reaktivität sekundärer heteroatomsubstituierter Amine kann auch durch eine gegenüber primären heteroatomsubstituierten Aminen erhöhte Menge an Katalysator ausgeglichen werden.

Pro Mol umzusetzendes Amin werden 0,5 bis 3 Mol Ester zugesetzt. Auch bei Verwendung racemischer Substrate werden 0,5 bis 3, bevorzugt 0,5 bis 1,0 Mol Ester zugesetzt.

Die zuzusetzende Menge an Enzym hängt von der Art der Hydrolase und der Aktivität der Enzympräparation ab. Die für die Reaktion optimale Enzymmenge kann leicht durch einfache Vorversuche ermittelt werden. In der Regel werden 1000 Units Lipase pro mMol heteroatomsubstituierten Amin zugesetzt.

Der Reaktionsverlauf läßt sich leicht mit üblichen Methoden beispielsweise mittels Gaschromatographie verfolgen. Im Falle der Racematspaltung beendet man die Reaktion sinnvollerweise bei einem Umsatz von 50% des racemischen heteroatomsubstituierten Amins. Dies geschieht in der Regel durch Entfernen des Katalysators aus dem Reaktionsraum, beispielsweise durch Abfiltrieren des Enzyms.

Durch die enantioselektive Umsetzung des racemischen Substrats mit dem Ester entsteht aus dem einen Enantiomer das entsprechend acylierte Produkt (Amid), während das andere Enantiomer unverändert bleibt. Das nun vorliegende Gemisch aus heteroatomsubstituiertem Amin und Amid läßt sich mit üblichen Methoden leicht trennen. Gut geeignet zur Trennung des Gemisches aus Amin und Amid sind beispielsweise Extraktions- oder Destillationsverfahren.

Das erfindungsgemäße Verfahren eignet sich besonders vorteilhaft zur Acylierung von heteroatomsubstituierten Aminen der Formel I. Auch die Racematspaltung von praktisch allen primären und sekundären heteroatomsubstituierten Aminen ist damit durchführbar. Besonders gut verläuft es bei primären Aminoalkoholen, vor allem solchen, bei denen R⁴ Arylalkyl, insbesondere Benzyl, oder Alkyl, insbesondere Methyl bedeutet.

Weitere bevorzugte Verbindungen der Formel I sind solche, bei denen R¹ und R² mit den benachbarten C-Atomen ein Ringsystem bilden, insbesondere solche der folgenden Struktur 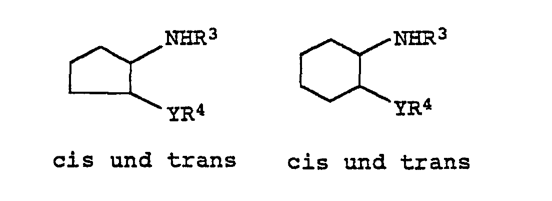 oder R² und R³ Teil eines Ringsystems sind, insbesondere solche der folgenden Struktur 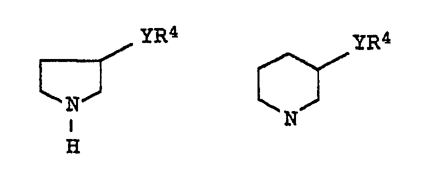 oder R¹ und R⁴ Teil eines Ringsystems sind, insbesondere solche der folgenden Struktur

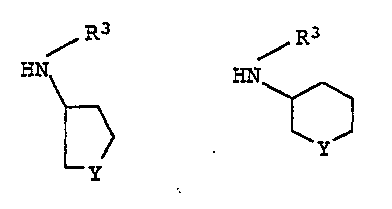

Überraschenderweise verläuft die Umsetzung von heteroatomsubstituierten Aminen der Formel I mit sehr viel höheren optischen Ausbeuten als die analoge Umsetzung nicht-heteroatomsubstituierter bzw. anders als in Formel I substituierter Amine.

Weiterhin bedarf es infolge der hohen Selektivität und Reaktivität des erfindungsgemäßen Verfahrens keines oder nur eines geringen Überschusses an Acylierungsmittel, was die nachfolgende Trennung und Reinigung stark erleichtert.

Die Erfindung eignet sich auch zur Herstellung von optisch aktiven primären und sekundären heteroatomsubstituierten Aminen aus den entsprechenden Racematen, in dem man
a) ein racemisches heteroatomsubstituiertes Amin mit einem Ester, dessen Säurekomponente ein Sauerstoffatom trägt, das an ein Kohlenstoffatom in alpha-Position zum Carbonylkohlenstoffatom gebunden ist, in Gegenwart einer Lipase ausgewählt aus der Gruppe SP 523, SP 524, SP 525, SP 526 oder Novozym® 435 enantioselektiv acyliert,
b) das Gemisch aus optisch aktivem heteroatomsubstituiertem Amin und optisch aktivem acyliertem heteroatomsubstituiertem Amin trennt und somit ein Enantiomer des heteroatomsubstituierten Amins erhält,
c) gewünschtenfalls aus dem acylierten heteroatomsubstituierten Amin das andere Enantiomere des heteroatomsubstituierten Amins durch Amidspaltung gewinnt.

Das erfindungsgemäße Verfahren läßt sich noch ökonomischer gestalten, wenn man nach Abtrennung des gewünschten Enantiomers das verbliebene, nicht gewünschte Enantiomer racemisiert und erneut in das Verfahren einsetzt. Durch diese Rückführung wird es möglich, insgesamt mehr als 50% des gewünschten Enantiomers aus dem racemischen heteroatomsubstituierten Amin zu erhalten.

Die erfindungsgemäßen Verfahren eignen sich nicht nur als Herstellverfahren zur Produktion optisch aktiver primärer und sekundärer heteroatomsubstituierten Amine, sondern können auch Bestandteil von komplizierten chemischen Mehrstufensynthesen, beispielsweise bei der Herstellung von Arzneiwirkstoffen oder Pflanzenschutzmitteln, sein.

Die folgenden Beispiele dienen der Veranschaulichung der Erfindung.

### Beispiel 1: Allgemeine Arbeitsvorschrift Lipase-katalysierte Acylierung von heteroatomsubstituierten Aminen

10 mmol des primären oder sekundären heteroatomsubstituierten Amins werden in MTBE (= Methyl-tert.butylether) gelöst (ca. 10 %ige Lösung). Die Lösung wird mit 11 mmol Methoxyessigsäureethylester versetzt und die Reaktion durch Zusatz von 100 mg Lipase (ca. 1000 U/mg, Pseudomonas spec. DSM 8246) gestartet. Bei vollständigem Umsatz (je nach heteroatomsubstituierten Aminen 12 bis 48 h) wird das Enzym abfiltriert und die Lösung im Vakuum konzentriert. Man erhält die Methoxyacetamide in einer Ausbeute von über 90 Prozent.

### Beispiel 2: Allgemeine Arbeitsvorschrift für Racematspaltung

Das primäre oder sekundäre heteroatomsubstituierten Aminen wird in MTBE gelöst (ca. 10 Gew.-%). Nach Zusatz von 1 Mol Methoxyessigsäureethylester pro 1 Mol racemisches heteroatomsubstituiertes Amin wird Pseudomonas-Lipase (DSM 8246) zugesetzt und die Suspension bei Raumtemperatur gerührt. Pro mMol heteroatomsubstituiertes Amin werden etwa 10000 Units Lipase (10mg) zugesetzt. Nach Erreichen eines Umsatzes von 50 % (Überprüfung mittels Gaschromatographie), was je nach heteroatomsubstituierten Aminen nach 1 bis 48 h erreicht ist, wird das Enzym abfiltriert. Das Gemisch aus heteroatomsubstituierten Aminen und acyliertem heteroatomsubstituiertem Amin (Amid) wird durch Destillation oder Extraktion getrennt.

### Beispiel 3: Racematspaltung mit Lösungsmittel

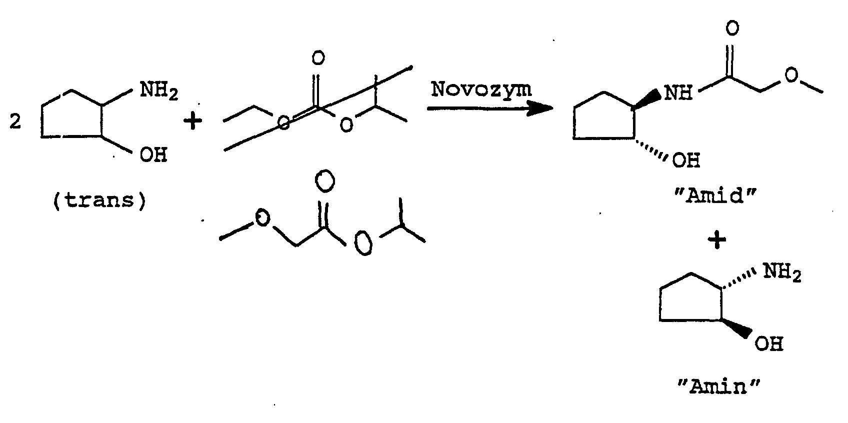

5 g (49,5 mmol) trans-2-Aminocyclopentanol wurden in 20 ml 1,4-Dioxan gelöst, mit 3,3 g (25 mmol) Methoxyessigsäure-isopropylester versetzt und nach Zugabe von 0,1 g Novozym 435® bei Raumtemperatur geschüttelt. Nach 12 h war laut ¹H-NMR 50 % des eingesetzten Amins umgesetzt; man filtrierte das Enzym ab, engte das Filtrat ein und trennte das unumgesetzte Amin destillativ vom gebildeten Amid ab.

### Ausbeuten:

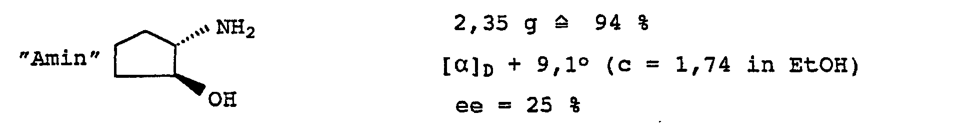

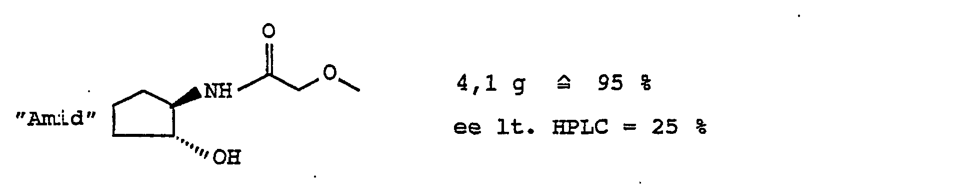

### Beispiel 4: Racematspaltung ohne Lösungsmittel

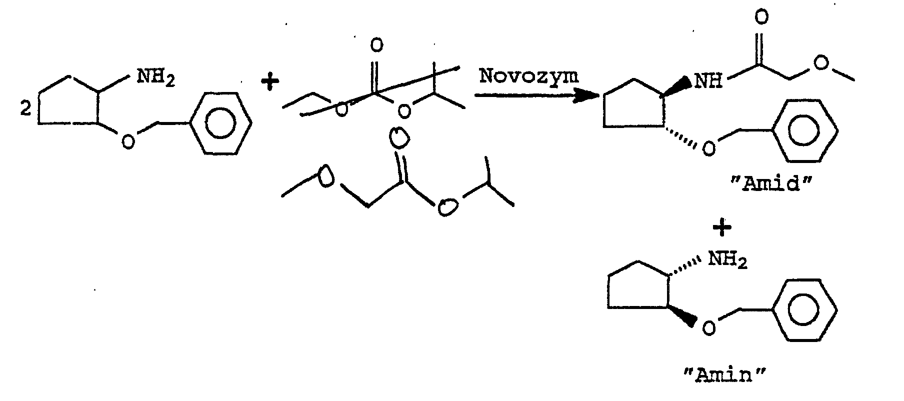

5 g (26 mmol) trans-2-Benzyloxy-1-cyclopentylamin und 1,8 g (13,4 mmol) Methoxyessigsäure-isopropylester wurden gemischt, mit 0,1 g Novozym 435® versetzt und bei Raumtemperatur geschüttelt. Lt. ¹H-NMR waren nach 120 h 50 % des Amins umgesetzt. Das Enzym wurde abfiltriert und das "Amin" durch Extraktion mit 10 %iger Salzsäure vom "Amid" abgetrennt.

### Ausbeuten:

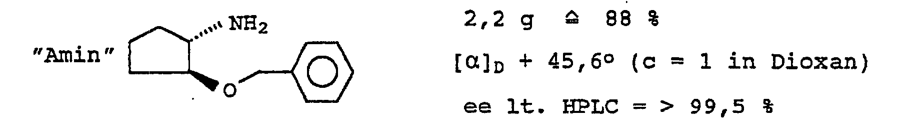

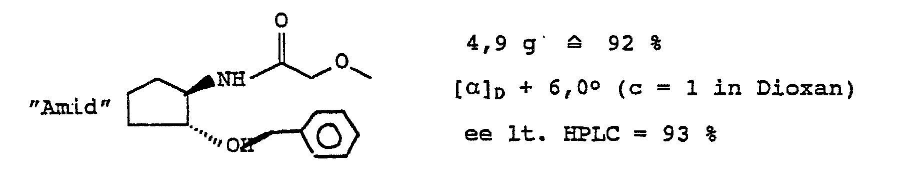

### Beispiel 5: Weitere Racematspaltungen

Gemäß Beispiel 3 bzw. 4 wurden folgende Umsetzungen (s. Tabelle) durchgeführt.

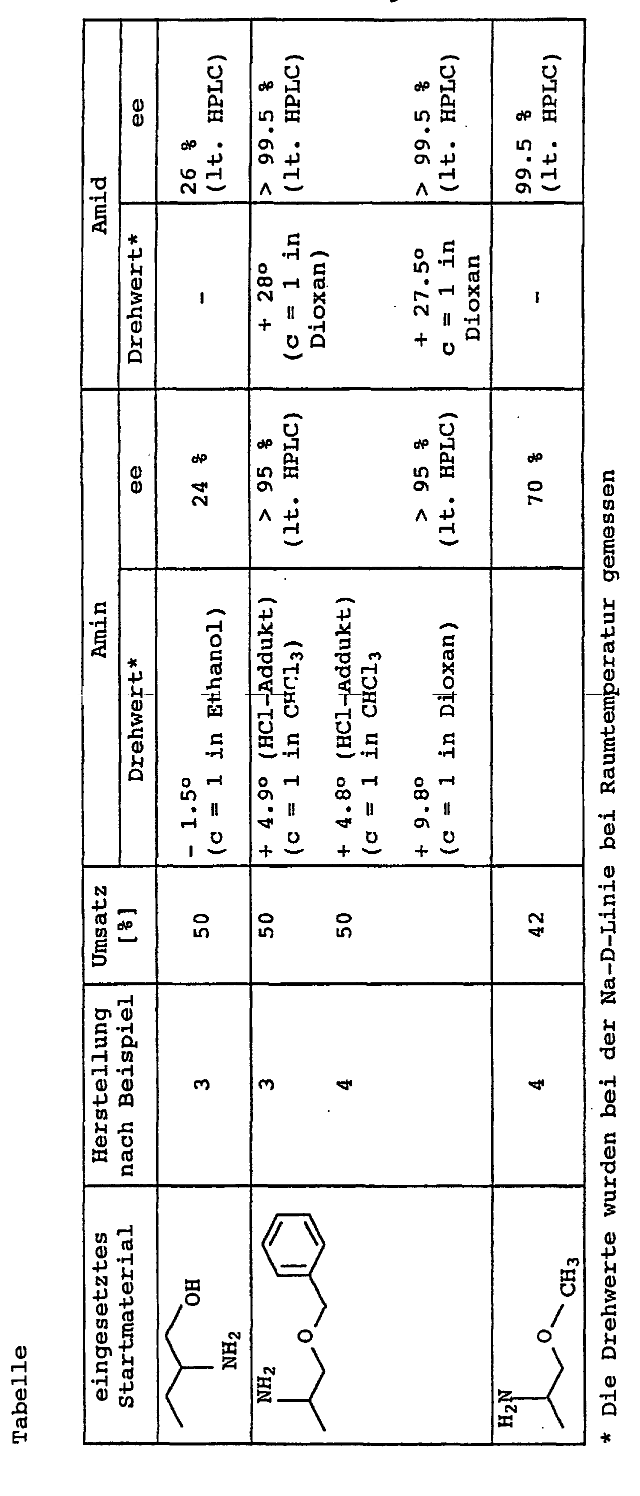

Die Tabelle in Beispiel 5 zeigt, daß bei Verwendung "geschützter" Aminoalkohole, bei denen das Sauerstoffatom beispielsweise in Nachbarschaft zu einer Benzyl- oder Methylgruppe steht, sich sehr viel höhere optische Reinheiten erzielen lassen als bei Verwendung ungeschützter Aminoalkohole.

## Patentansprüche

1. Verfahren zur Herstellung von acylierten primären und sekundären heteroatomsubstituierten Aminen durch Umsetzung der heteroatomsubstituierten Amine mit einem Ester in Gegenwart einer Lipase aus Hefe ausgewählt aus der Gruppe SP 523, SP 524, SP 525, SP 526 oder Novozym® 435, wobei die Säurekomponente des Esters ein Sauerstoffatom trägt, das an ein Kohlenstoffatom in alpha-Position zum Carbonylkohlenstoff gebunden ist.

2. Verfahren zur Racematspaltung primärer und sekundärer heteroatomsubstituierten Amine durch Umsetzung mit einem Ester in Gegenwart einer Lipase aus Hefe ausgewählt aus der Gruppe SP 523, SP 524, SP 525, SP 526 oder Novozym® 435 und anschließende Trennung des einen, enantioselektiv acylierten heteroatomsubstituierten Amins vom nicht umgesetzten anderen Enantiomer des heteroatomsubstituierten Amins, wobei die Säurekomponente des Esters ein Sauerstoffatom trägt, das an ein Kohlenstoffatom in alpha-Position zum Carbonylkohlenstoff gebunden ist.

3. Verfahren zur Herstellung von optisch aktiven primären und sekundären heteroatomsubstituierten Aminen aus den entsprechenden Racematen, indem man
a) ein racemisches heteroatomsubstituiertes Amin mit einem Ester, dessen Säurekomponente ein Sauerstoffatom trägt, das an ein Kohlenstoffatom in alpha-Position zum Carbonylkohlenstoff gebunden ist, in Gegenwart einer Lipase aus Hefe ausgewählt aus der Gruppe SP 523, SP 524, SP 525, SP 526 oder Novozym® 435 enantioselektiv acyliert,
b) das Gemisch aus optisch aktivem heteroatomsubstituiertem Amin und optisch aktivem acylierten heteroatomsubstituierten Amin trennt und somit ein Enantiomer des heteroatomsubstituierten Amins erhält,
c) gewünschtenfalls aus dem acylierten heteroatomsubstituierten Amin das andere Enantiomere des Amins durch Amidspaltung gewinnt.

4. Verfahren gemäß Anspruch 3, **dadurch gekennzeichnet, daß** man in Anschluß an Schritt b) oder c) in einem weiteren Schritt das nicht-gewünschte Enantiomer racemisiert und anschließend in das Verfahren der Racematspaltung zurückführt.

5. Verfahren zur Herstellung von optisch aktiven Verbindungen, **dadurch gekennzeichnet, daß** es mindestens als Teilschritt ein Verfahren nach Anspruch 2 bis 4 beinhaltet.

6. Verfahren nach Anspruch 2 oder 3, **dadurch gekennzeichnet, daß** als primäres oder sekundäres heteroatomsubstituiertes Amin ein Amin der allgemeinen Formel I 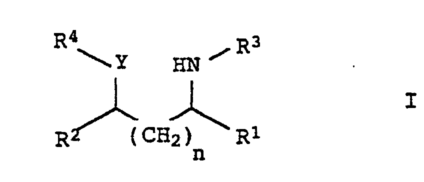 wobei
n 0, 1, 2, 3;
Y 0, S, NH, NR⁵;
R¹,R² unabhängig voneinander jeweils H, Alkyl oder Aryl oder R¹ und R², oder R² und R³, oder R¹ und R⁴ zusammen mit den benachbarten C-Atomen Teil eines Ringsystems;
R⁴ Alkyl oder Arylalkyl;
R³,R⁵ unabhängig voneinander H, Alkyl oder Arylalkyl bedeuten,
eingesetzt wird.

7. Verfahren nach Anspruch 6, **dadurch gekennzeichnet, daß** Verbindungen der Formel I mit Y = O eingesetzt werden.

8. Verfahren nach Anspruch 7, **dadurch gekennzeichnet, daß** R⁴ die Bedeutung Methyl oder Benzyl hat.

9. Verfahren nach einem der vorstehenden Ansprüche, **dadurch gekennzeichnet, daß** als Ester ein Methoxyessigsäurealkylester verwendet wird.

## Claims

1. A process for preparing acylated primary and secondary heteroatom-substituted amines by reacting the heteroatom-substituted amines with an ester in the presence of a lipase from yeast selected from the group of SP 523, SP 524, SP 525, SP 526 or Novozym® 435, wherein the acid component of the ester carries an oxygen atom which is bonded to a carbon atom in the position alpha to the carbonyl carbon.

2. A process for resolving racemates of primary and secondary heteroatom-substituted amines by reaction with an ester in the presence of a lipase from yeast selected from the group of SP 523, SP 524, SP 525, SP 526 or Novozym® 435 and subsequent separation of the heteroatom-substituted amine which has undergone enantioselective acylation from the other, unreacted, enantiomer of the heteroatom-substituted amine, wherein the acid component of the ester carries an oxygen atom which is bonded to a carbon atom in the position alpha to the carbonyl carbon.

3. A process for preparing optically active primary and secondary heteroatom-substituted amines from the corresponding racemates, by
a) enantioselective acylation of a racemic heteroatom-substituted amine with an ester whose acid component carries an oxygen atom which is bonded to a carbon atom in the position alpha to the carbonyl carbon, in the presence of a lipase from yeast selected from the group of SP 523, SP 524, SP 525, SP 526 or Novozym® 435,
b) separation of the mixture of optically active heteroatom-substituted amine and optically active acylated heteroatom-substituted amine to obtain one enantiomer of the heteroatom-substituted amine,
c) if required isolation of the other enantiomer of the amine from the acylated heteroatom-substituted amine by amide cleavage.

4. A process as claimed in claim 3, wherein step b) or c) is followed by another step in which the unwanted enantiomer is racemized and subsequently returned to the racemate resolution process.

5. A process for preparing optically active compounds, in which at least one step comprises a process as claimed in claim 2 to 4.

6. A process as claimed in claim 2 or 3, wherein the primary or secondary heteroatom-substituted amine used is an amine of the general formula I 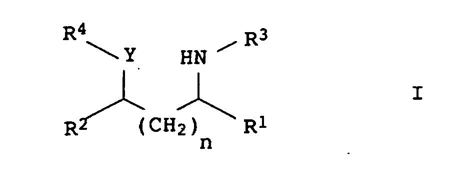 where
n is 0, 1, 2, 3;
Y is O, S, NH, NR⁵;
R¹, R² are each, independently of one another, H, alkyl or aryl or R¹ and R², or R² and R³, or R¹ and R⁴ are, together with the adjacent carbon atoms, part of a ring system;
R⁴ is alkyl or arylalkyl;
R³, R⁵ are, independently of one another, H, alkyl or arylalkyl.

7. A process as claimed in claim 6, wherein compounds of the formula I with Y = O are used.

8. A process as claimed in claim 7, wherein R⁴ is methyl or benzyl.

9. A process as claimed in any of the preceding claims, wherein the ester used is an alkyl methoxyacetate.

## Revendications

1. Procédé pour la préparation d'amines primaires et secondaires à substituants contenant des hétéroatomes et acylées par réaction des amines à substituants contenant des hétéroatomes avec un ester en présence d'une lipase de levure choisie dans le groupe consistant en SP 523, SP 524, SP 525, SP 526 et Novozym 435, le composant acide de l'ester portant un atome d'hydrogène fixé sur un atome de carbone en position alpha du carbone de carbonyle.

2. Procédé pour la résolution de racémates d'amines primaires et secondaires à substituants contenant des hétéroatomes par réaction avec un ester en présence d'une lipase de levure choisie dans le groupe consistant en SP 523, SP 524, SP 525, SP 526 et Novozym 435 suivie de la séparation entre l'une des amines à substituants contenant des hétéroatomes, qui a subi une acylation énantiosélective, et l'autre énantiomère de l'amine à substituants contenant des hétéroatomes, qui n'a pas été converti, le composant acide de l'ester portant un atome d'oxygène fixé sur un atome de carbone en position alpha du carbone de carbonyle.

3. Procédé pour la préparation d'amines primaires et secondaires à substituants contenant des hétéroatomes à l'état d'isomères optiques à partir des racémates correspondants, dans lequel
a) on soumet une amine à substituants contenant des hétéroatomes, à l'état de racémique, à acylation énantiosélective par un ester dont le composant acide porte un atome d'oxygène fixé sur un atome de carbone en position alpha du carbone de carbonyle en présence d'une lipase de levure choisie dans le groupe consistant en SP 523, SP 524, SP 525, SP 526 et Novozym 435,
b) on sépare le mélange de l'amine à substituants contenant des hétéroatomes, à l'état d'isomère optique et de l'amine à substituants contenant des hétéroatomes, acylée, à l'état d'isomère optique, ce qui donne un énantiomère de l'amine à substituants contenant des hétéroatomes,
c) si on le désire, à partir de l'amine à substituants contenant des hétéroatomes, acylée, on obtient l'autre énantiomère de l'amine par scission de l'amide.

4. Procédé selon la revendication 3, **caractérisé par le fait que**, à la suite du stade b) ou du stade c), dans un autre stade opératoire, on racémise l'énantiomère non recherché et on le recycle à l'opération de résolution du racémate.

5. Procédé pour la préparation d'isomères optiques, **caractérisé par le fait que** l'on applique un procédé selon les revendications 2 à 4 au moins en tant que stade partiel.

6. Procédé selon la revendication 2 ou 3, **caractérisé par le fait que** l'on met en oeuvre, en tant qu'amine primaire ou secondaire à substituants contenant des hétéroatomes, une amine de formule générale I 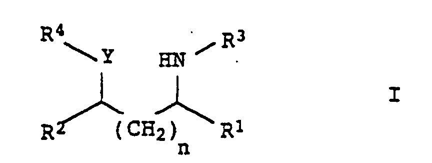 dans laquelle
n est égal à 0, 1, 2, 3 ;
Y représente O, S, NH, NR⁵ ;
R¹, R² représentent chacun, indépendamment l'un de l'autre, H, un groupe alkyle ou aryle, ou bien R¹ et R², ou R² et R³, ou R¹ et R⁴ représentent ensemble et avec les atomes de carbone voisins, une partie d'un système cyclique ;
R⁴ représente un groupe alkyle ou arylalkyle ;
R³, R⁵ représentent chacun, indépendamment l'un de l'autre, H, un groupe alkyle ou arylalkyle.

7. Procédé selon la revendication 6, **caractérisé par le fait que** l'on met en oeuvre des composés de formule I dans laquelle Y = O.

8. Procédé selon la revendication 7, **caractérisé par le fait que** R⁴ représente un groupe méthyle ou benzyle.

9. Procédé selon l'une des revendications qui précèdent, **caractérisé par le fait que** l'ester utilisé est un méthoxyacétate d'alkyle.
